# EUROPEAN PATENT SPECIFICATION

(11) **EP 0 611 962 B1**
(45) Date of publication and mention of the grant of the patent: **29.04.1998**
(21) Application number: 94830063.7
(22) Date of filing: 18.02.1994
(51) Int. Cl.: G01N 1/24

(54) **Method and apparatus for extracting particulate from the exhaust gases of diesel engines**
Verfahren und Vorrichtung zur Extraktion von Teilchen aus Dieselmotorabgas
Méthode et dispositif pour l'extraction de particules des gaz d'échappement de moteurs diesel

(30) Priority: 19.02.1993 IT TO930105
(43) Date of publication of application: 24.08.1994
(73) Proprietor: IVECO FIAT S.p.A., 10156 Torino (IT); CONTROL SISTEM S.r.l., I-10044 Pianezza (Torino) (IT)
(72) Inventor: d'Angelo, Lorenzo, I-10090 Rivoli (Torino) (IT); Roccatello, Graziano, I-10095 Grugliasco (Torino) (IT); Appino, Antonio, I-10080 Rivara (Torino) (IT)
(74) Representative: Notaro, Giancarlo

(56) References cited:
- EP-A- 0 388 392
- EP-A- 0 471 174
- WO-A-92/04611
- DE-A- 4 018 872
- DE-A- 4 121 928
- GB-A- 2 214 449
- PATENT ABSTRACTS OF JAPAN vol. 15, no. 344 (P-1245) 30 August 1991 & JP-A-03 130 640 (HINO MOTORS LTD.) 4 June 1991

## Description

The present invention relates to the methods and apparatus for measuring the quantity of particulate contained in exhaust gasses of Diesel internal combustion engines. Said apparatus are necessary in order to carry out tests provided by the laws for homologation of Diesel engines for motor-vehicles.

The rules in force in the United States provide that the engine is run through a working cycle simulating actual traffic conditions. According to the test provided by these rules, the entire exhaust gas flow is diluted with ambient air in a dilution tunnel. The entire diluted gas flow is kept constant, by adjusting the dilution ratio when exhaust gas flow increases or decreases. After mixing, a sample of the diluted flow is extracted and passed through a filter which collects the particulate. The quantity of extracted particulate is than determined by weighing.

The main drawback of the above indicated method lies in that it implies the use of apparatus of huge dimension and very high cost. For this reason, said system with total dilution of the exhaust gas flow, called also CVS (Constant Volume Sampling) is not fully satisfactory from the point of view of convenience and economy of use.

Until 1991, the test adopted in the EEC did not provide for the measuring of the quantity of particulate, but only an opacimeter measurement of the quantity of light transmitted through the undiluted exhaust gas flow.

Starting from 1992, the EEC rule has introduced a gravimetric measurement of the particulate, accepting the use of a partial dilution system of the exhaust gasses. According to such system, most part of the exhaust gas flow is not used for the test, whereas a portion thereof is extracted to be diluted with ambient air for the following gravimetric determination of the quantity of particulate contained therein.

There have been proposed already methods and apparatus for extracting the particulate from the exhaust gasses according to the partial dilution system. The advantage of the methods of this type is that they use apparatus which are much less bulky and less costly with respect to those necessary for the CVS tests. However, the apparatus used up to now for conducting tests of the partial dilution type are not fully satisfactory, basically for their insufficient precision compared with the results which may be obtained with the CVS systems.

An apparatus having the features indicated in the pre-characterizing portion of Claim 1 is known from WO-A-9204611. However this known apparatus basically operates under a constant dilution ratio.

The object of the present invention is to provide an apparatus for carrying out a particulate sampling method of the partial dilution type which gives a more precise measurement with respect to the partial dilution methods used to date.

In order to achieve this object, the invention provides an apparatus having the features specified in the annexed Claim 1. The invention also provides a method as set forth in Claim 2.

The main feature of the apparatus according to the invention lies in that it provides precision volumetric meters arranged to measure directly the volumetric flow of the dilution air and the diluted gas. The apparatus also comprises precision meters for the temperature and pressure of the dilution air and the diluted gas as well as a computer to process the signals emitted by said meters and to provide an indication of the flows of the dilution air and the diluted gas. The exhaust gas flow is obtained by difference, so as to avoid any interference with the flows of the exhaust gasses. The computer is also arranged to determine at each instant the total volume of gas which has flowed so as to interrupt the passage of the gas through the filter when a predetermined total gas volume has been reached. Due to said features, it is possible to extract samples of particulate not only in a determined time interval, but also on the basis of a predetermined volume of exhaust gas.

The dilution tunnel is mounted on a movable carriage which can be easily and rapidly moved and which is provided which a motorised pinion-and-rack system for quick positioning of the exhaust gas extracting probe.

In order to keep the diluted gas flow constant also during stages of the test when the gas is not passed through the filter, there is provided a by-pass conduit in parallel to the filter, in which a regulation valve is interposed. An electronic circuit attends to adjusting the pump for sucking the diluted gas in order to provide the above indicated constant flow condition.

Further features and advantages of the invention will become apparent from the description which follows with reference to the annexed drawing, given purely by way of non limiting example, which shows a diagram of the apparatus according to the invention.

In the diagram illustrated in the annexed drawing, reference numeral 1 designates the tube which receives the entire exhaust gas flow of a Diesel engine, from which a portion is extracted by the probe designated with reference numeral 2. Probe 2 leads the extracted portion of the exhaust gas flow within a tunnel 3 having an initial portion 4 for mixing the exhaust gasses with atmospheric air coming through conduit 5. The atmospheric air reaches the initial portion 4 of tunnel 3 through conduit 5, pushed by a pump 6 which sucks air from an inlet mouth 7 through a filter 8. Pressure and temperature of the air fed to conduit 5 are measured with precision by precision meters 9, 10. Reference numeral 11 designates a volumetric flow meter arranged in conduit 5 upstream of the pump 6. The gas diluted in tunnel 3 exits through an outlet end 12 to which a filter 13 is connected (which in the preferred embodiment is constituted by a unit including two filters in series) which is to collect the particulate contained in the diluted gas. The outlet of the filter is connected to a conduit 14 in which there are interposed two heat-exchangers 15, 16 (for keeping the gas temperature constant at the inlet of a volumetric flow meter 22), pressure and temperature precision meters 17, 18, 19, 20, a pump 21 controlled by a regulating system 29 and a volumetric flow meter 22 arranged immediately upstream of the outlet 23 for the gas. In parallel to filter 13 there is provided a by-pass conduit 24 in which there are interposed a filter 25, and a motorised regulation valve 26. Two solenoid valves 27, 28 are also interposed in conduit 14 and in conduit 24. Valve 26 can be regulated so as to simulate the loss in load due to the progressive clogging of filter 13 during the stages of the test in which the gasses are passed through the by-pass conduit 24 rather than through filter 13.

All the components contained within the dotted ring indicated by letter A in the annexed diagram are mounted on a carriage which can be rapidly moved and is provided with a motorised pinion-and-rack system (not illustrated) for vertically positioning the probe 2. The remaining components are contained in a control cabinet.

The apparatus further includes a computer which receives the signal outputted by the volumetric flow meters so as to compute in real time the values of volumetric flow and volume of the diluted gas and the dilution air and, by difference, the total flow of exhaust gasses.

The computer is programmed to compare, instant by instant, a predetermined value of volume of exhaust gas with the progressive value of volume computed by integration of all the values read up to that moment, so as to check when the said predetermined value of volume is reached and, as a consequence of this, cause the switching of the passage of the diluted gasses from conduit 15 to conduit 24 by shutting of valve 28 and opening valve 27. At the end off the test, the particulate collected in filter 13 is determined by weighing.

As already indicated above, by the features which have been illustrated it is possible to carry out measurements more precise with respect to those which can be obtained with the apparatus of the partial dilution type which have been used up today.

Naturally, the principle of the invention remaining the same, the construction details and the embodiments may amply vary with respect to what have been described and illustrated purely by way of example, without departing from the scope of the present invention.

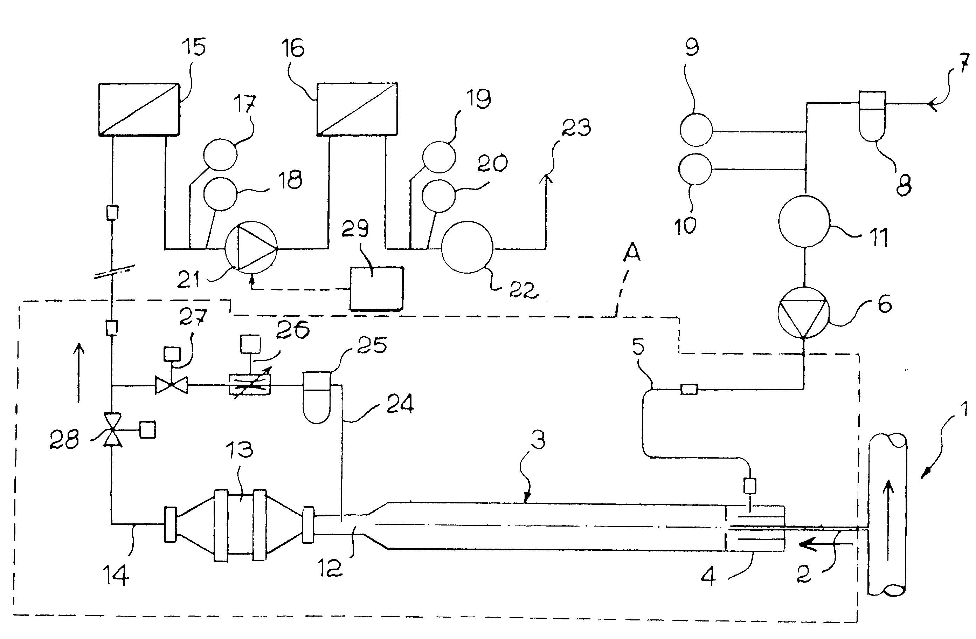

## Claims

1. Apparatus for extracting particulate from the exhaust gasses of an internal combustion Diesel engine, wherein :
- the apparatus comprises an exhaust gas dilution tunnel provided with a probe for feeding a portion of the exhaust gas flow coming from the engine into a dilution tunnel,
- said probe (2) is communicated to an inlet portion (4) of the dilution tunnel (3) in which a conduit (5) for feeding dilution atmospheric air opens,
- in said conduit (5) for feeding dilution air there are interposed a pressure meter (9), a temperature meter (10) and a volumetric flow meter (11),
- the outlet of the dilution tunnel (3) is communicated to a filter (13) for collecting particulate contained in the diluted gas, the outlet of the filter (13) being connected to a conduit (14) in which there are interposed at least a pressure meter (19), a temperature meter (20) and a volumetric flow meter (22), characterized in that said apparatus comprises:
- a pump (6) arranged in said conduit (5) for feeding dilution air,
- a computer which receives output signals from said volumetric flow meters and is arranged to determine the volumetric flow of diluted gas and dilution air and, by difference, of the exhaust gasses, as well as to compute, instant by instant, the volume of flowed gas, and to compare the latter with a predetermined value of volume, so as to check when the volume of flowed gas is equal to said predetermined value, and in that in parallel to filter (13) there is arranged a by-pass conduit (24) in which there is interposed a regulation valve (26), and in that valve means are provided to address selectively the diluted gas to said filter (13) or to said by-pass conduit (24), as well as control means for regulating a pump (21) for sucking the diluted gas so as to keep the gas flow constant during all the stages of the test.

2. Method for extracting particulate contained in the exhaust gasses of an internal combustion Diesel engine, wherein :
- a portion of the exhaust gas flow from the engine is extracted,
- the extracted portion of the exhaust gas flow is fed into a dilution tunnel (3) together with a flow of dilution atmospheric air,
- the diluted gas flow coming from the dilution tunnel (3) is fed to a filter (13) to collect the particulate contained in the gas,
- pressure and temperature of the dilution air and diluted gas are measured,
- volumetric flow of the dilution air and the diluted gas are measured, characterized in that said flow of dilution atmospheric air is created by a pump (6), in that said measurements are processed by an electronic computer so as to obtain the measurement of the volumetric flow of the dilution air and the diluted gas and, by difference, of the exhaust gasses as well as to determine, instant by instant, the volume of flowed gas, so as to check when a predetermined value of volume is reached, and in that when the reaching of said predetermined value of volume is sensed, the flow of diluted gas is addressed into a by-pass conduit (24) parallel to the filter (13) and including a regulation valve (26), and the pump for sucking the diluted gas is adjusted so as to keep the gas flow constant.

## Patentansprüche

1. Vorrichtung zum Extrahieren von Partikelmaterial aus den Abgasen eines Diesel-Verbrennungsmotors, wobei:
- die Vorrichtung einen Abgas-Verdünnungstunnel aufweist, welcher mit einer Sonde zum Einleiten eines Teils des von dem Motor kommenden Abgasstromes in einen Verdünnungstunnel ausgestattet ist,
- diese Sonde (2) mit einem Einlaßabschnitt (4) des Verdünnungstunnels (3) in Verbindung gebracht ist, in welchen sich eine Leitung (5) zum Zuführen atmosphärischer Verdünnungsluft öffnet,
- in der genannten Leitung (5) zum Einführen von Verdünnungsluft ein Druckmeßgerät (9), ein Temperaturmeßgerät (10) und ein Meßgerät (11) für einen volumetrischen Strom eingefügt sind,
- der Auslaß des Verdünnungstunnels (3) mit einem Filter (13) zum Sammeln von Partikelmaterial in Verbindung gebracht ist, das in dem verdünnten Gas enthalten ist, wobei der Auslaß des Filters (13) mit einer Leitung (14) verbunden ist, in welcher wenigstens ein Druckmeßgerät (19), ein Temperaturmeßgerät (20) und ein Meßgerät (22) für einen volumetrischen Strom eingefügt sind,
**dadurch gekennzeichnet**, daß die Vorrichtung folgendes aufweist:
- eine in der Leitung (5) angeordnete Pumpe (6) zum Fördern von Verdünnungsluft,
- einen Computer, welcher Ausgabesignale von den Meßgeräten für einen volumetrischen Strom aufnimmt und so ausgebildet ist, daß er den volumetrischen Strom von verdünntem Gas und Verdünnungsluft sowie, durch Differenzbildung, der Abgase bestimmt und daß er Augenblick für Augenblick das Volumen des geströmten Gases berechnet und das letztere mit einem vorgegebenen Volumenwert vergleicht, um zu prüfen, wann das Volumen des geströmten Gases gleich dem vorgegebenen Wert ist,
und daß parallel zu dem Filter (13) eine Bypass-Leitung (24) angeordnet ist, in welcher ein Regelventil (20) eingefügt ist, und daß Ventilmittel vorgesehen sind, um wahlweise das verdünnte Gas zu dem Filter (13) oder zu der Bypass-Leitung (24) zu leiten, sowie Steuermittel zum Regulieren einer Pumpe (21) zum Ansaugen des verdünnten Gases, um den Gasstrom während aller Stufen des Testes konstant zu halten.

2. Verfahren zum Extrahieren von in den Abgasen eines Diesel-Verbrennungsmotors enthaltenem Partikelmaterial, wobei:
- ein Teil des Abgasstromes von dem Motor abgezweigt wird,
- der abgezweigte Teil des Abgasstromes zusammen mit einem Strom von atmosphärischer Verdünnungsluft in einen Verdünnungstunnel (3) geleitet wird,
- der von dem Verdünnungstunnel (3) kommende verdünnte Gasstrom einem Filter (13) zugeführt wird, um das in dem Gas enthaltene Partikelmaterial zu sammeln,
- Druck und Temperatur der Verdünnungsluft und des verdünnten Gases gemessen werden,
- der volumetrische Strom der Verdünnungsluft und des verdünnten Gases gemessen werden,
**dadurch gekennzeichnet**, daß der Strom der atmosphärischen Verdünnungsluft durch eine Pumpe (6) erzeugt wird, daß die Messungen durch einen elektronischen Computer verarbeitet werden, um so die Messung des volumetrischen Stromes der Verdünnungsluft und des verdünnten Gases und, durch Differenzbildung, der Abgase zu gewinnen, und gleichzeitig Augenblick für Augenblick das Volumen des geströmten Gases zu bestimmen, um so zu prüfen, wann ein vorgegebener Wert des Volumens erreicht wird, und daß dann, wenn das Erreichen dieses vorgegebenen Volumenwertes erfaßt wird, der Strom des verdünnten Gases in eine Bypass-Leitung (24) geleitet wird, welche parallel zu dem Filter (13) angeordnet ist und ein Regelventil (26) umfaßt, und daß die Pumpe zum Ansaugen des verdünnten Gases so eingestellt wird, daß sie den Gasstrom konstant hält.

## Revendications

1. Appareil d'extraction de particules des gaz d'échappement d'un moteur à combustion interne diesel, dans lequel :
- l'appareil comporte un tunnel de dilution de gaz d'échappement ayant une sonde destinée à transmettre une partie du courant de gaz d'échappement provenant du moteur à un tunnel de dilution,
- la sonde (2) communique avec une partie d'entrée (4) du tunnel de dilution (3) dans laquelle un conduit (5) de transmission d'air atmosphérique de dilution débouche,
- un appareil (9) de mesure de pression, un appareil (10) de mesure de température et un appareil (11) de mesure de débit volumique sont placés dans ledit conduit (5) de transmission d'air de dilution, et
- la sortie du tunnel de dilution (3) communique avec un filtre (13) destiné à collecter des particules contenues dans les gaz dilués, la sortie du filtre (13) étant raccordée à un conduit (14) dans lequel sont disposés au moins un appareil (19) de mesure de pression, un appareil (20) de mesure de température, et un appareil (22) de mesure de débit volumique, et
caractérisé en ce que l'appareil comprend :
une pompe (6) placée dans le conduit (5) de transmission d'air de dilution, et
un ordinateur qui reçoit des signaux de sortie des appareils de mesure de débit volumique et qui est destiné à déterminer le débit volumique du gaz dilué et de l'air de dilution et, par différence, des gaz d'échappement, et qui est aussi destiné à calculer, à tout moment, le volume des gaz écoulés et à comparer celui-ci à une valeur prédéterminée du volume pour déterminer le moment où le volume des gaz qui s'est écoulé est égal à la valeur prédéterminée, et en ce que
un conduit (24) de dérivation est monté en parallèle avec le filtre (13) et comporte une soupape (26) de régulation, et un dispositif à soupape est destiné à adresser sélectivement le gaz dilué vers le filtre (13) ou vers le conduit de dérivation (24), et un dispositif de commande est destiné à réguler une pompe (21) d'aspiration des gaz dilués afin que le débit de gaz reste constant dans toutes les étapes du test.

2. Procédé d'extraction de particules contenues dans les gaz d'échappement d'un moteur à combustion interne diesel, dans lequel :
- une partie du courant de gaz d'échappement du moteur est extraite,
- la partie extraite du courant de gaz d'échappement est transmise à un tunnel de dilution (3) avec un courant d'air atmosphérique de dilution,
- le courant de gaz dilué provenant du tunnel de dilution (3) est transmis à un filtre (13) destiné à collecter les particules contenues dans le gaz,
- la pression et la température de l'air de dilution et du gaz dilué sont mesurées, et
- le débit volumique de l'air de dilution et celui du gaz dilué sont mesurés,
caractérisé en ce que le courant d'air atmosphérique de dilution est créé par une pompe (6), et en ce que
les mesures sont traitées par un ordinateur électronique pour l'obtention de la mesure des débits volumiques de l'air de dilution et du gaz dilué et, par différence, de celui des gaz d'échappement, et destiné aussi à déterminer, à tout moment, le volume des gaz qui se sont écoulés pour déterminer le moment où la valeur prédéterminée du volume est atteinte, et en ce que, lorsque le fait que la valeur prédéterminée du volume est atteinte a été détectée, le courant de gaz dilué est adressé à un conduit de dérivation (24) qui est monté en parallèle avec le filtre (13) et qui comporte une soupape de régulation (26), et la pompe d'aspiration du gaz dilué est ajustée afin qu'elle maintienne le débit de gaz à une valeur constante.
